(19) Europäisches Patentamt / European Patent Office / Office européen des brevets

(11) **EP 2 692 741 B1**

(12) **EUROPEAN PATENT SPECIFICATION**

(45) Date of publication and mention
of the grant of the patent:
**30.12.2015 Bulletin 2015/53**

(21) Application number: **12765809.4**

(22) Date of filing: **14.03.2012**

(51) Int Cl.:
*C08F 2/32* *(2006.01)*        *A61F 13/49* *(2006.01)*
*A61F 13/53* *(2006.01)*      *A61L 15/60* *(2006.01)*
*C08F 20/06* *(2006.01)*      *C08F 20/56* *(2006.01)*
*C08J 3/24* *(2006.01)*        *A61L 15/24* *(2006.01)*
*C08F 220/06* *(2006.01)*    *C08F 220/56* *(2006.01)*
*C08F 222/10* *(2006.01)*

(86) International application number:
**PCT/JP2012/056554**

(87) International publication number:
**WO 2012/132902 (04.10.2012 Gazette 2012/40)**

(54) **PROCESS FOR PRODUCING WATER-ABSORBING RESIN**

HERSTELLUNGSVERFAHREN FÜR WASSERABSORBIERENDES HARZ

PROCÉDÉ DE PRODUCTION D'UNE RÉSINE ABSORBANT L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2011 JP 2011078603**

(43) Date of publication of application:
**05.02.2014 Bulletin 2014/06**

(73) Proprietor: **Sumitomo Seika Chemicals Co., Ltd.
Kako-gun Hyogo 675-0145 (JP)**

(72) Inventor: **TAKATORI, Junichi
Hyogo 672-8076 (JP)**

(74) Representative: **Duckworth, Timothy John
J A Kemp
14 South Square
Gray's Inn
London WC1R 5JJ (GB)**

(56) References cited:
**EP-A1- 1 609 810        EP-A1- 2 011 803
WO-A1-2004/083284    WO-A1-2006/123561
WO-A1-2010/082373    JP-A- 9 012 613
JP-A- 2003 088 552**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a method for producing a water-absorbent resin, and a water-absorbent resin obtained thereby. More specifically, the present invention relates to a method for producing a water-absorbent resin according to reverse phase suspension polymerization, wherein the method gives a water-absorbent resin having excellent flow-through property, and a water-absorbent resin obtained thereby, and an absorbent material and an absorbent article using the water-absorbent resin.

BACKGROUND ART

Water-absorbent resins have been widely used in the recent years in various fields, including hygienic materials such as disposable diaper and sanitary napkin, horticultural materials such as water retaining materials and soil improvers, and industrial materials such as blocking materials and dew catchers. Among these fields, the water-absorbent resins are most often used especially in hygienic materials such as disposable diaper and sanitary napkin. As the water absorbent resins as mentioned above, for example, hydrolysates of starch-acrylonitrile graft copolymers, neutralized products of starch-acrylic acid graft polymers, saponified products of vinyl acetate-acrylic acid ester copolymers, crosslinked polymers of partially neutralized acrylic acid compound, and the like have been known.

Absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body. Usually, the absorbent material comprises a mixture of a water-absorbent resin and a hydrophilic fiber.

In the recent years, there have been increasing demands for thinning and light-weighing of absorbent articles, from the viewpoint of designing property and convenience upon carrying, and efficiency upon distribution. A method for thinning that is generally carried out in absorbent articles is, for example, a method of reducing hydrophilic fibers such as crushed pulp of a wood material, which has a role of fixing a water-absorbent resin in an absorbent material, while increasing a water-absorbent resin.

An absorbent material in which the proportion of a hydrophilic fiber which is large in volume and has low water absorption capacity is lowered, and a water-absorbent resin which is less in volume and high water absorption capacity is used in a large amount, is intended for thinning an absorbent material by reducing materials having large volumes, while obtaining absorption capacity matching the design of the absorbent article. However, when distribution or diffusion of a liquid upon actually using in an absorbent article such as disposable diapers is considered, there is a disadvantage that if a large amount of the water-absorbent resin is formed into a soft gel-like state by absorption, a so-called "gel-blocking phenomenon" takes place, whereby flow-through property is markedly lowered, and a liquid permeation time and liquid diffusibility of the absorbent material are worsened.

This "gel-blocking phenomenon" is a phenomenon in which especially when an absorbent material in which water-absorbent resins are highly densified absorbs a liquid, water-absorbent resins existing near a surface layer absorb the liquid to form soft gels that are even more densified near the surface layer, so that a liquid permeation into an internal of an absorbent material is inhibited, thereby making the internal of the water-absorbent resin incapable of efficiently absorbing the liquid. The larger the proportion of the fine powder in the water-absorbent resin, the more likely this phenomenon takes place. In view of the above, a water-absorbent resin having a high flow-through property after liquid absorption and swelling, in which gel blocking is less likely to take place, has been earnestly desired.

Water-absorbent resins have been primarily produced by subjecting a water-soluble ethylenically unsaturated monomer to a reversed phase suspension polymerization or an aqueous solution polymerization. However, in the conventional reversed phase suspension polymerization, there are some disadvantages that a water-absorbent resin having a large particle size is less likely to be produced, thereby making it less likely to obtain an appropriate particle size that is suitable for hygienic materials.

In view of the above, in order to obtain a water-absorbent resin having a large particle size, some production methods have been proposed. For example, a method for production according to a reversed phase suspension polymerization, including the steps of polymerizing first-step monomers to form a water-absorbent resin, cooling, again adding the monomer to a polymerization reaction mixture in which the first-step polymer particles are suspended in a state that a surfactant is precipitated to polymerize therewith, thereby obtaining a large water-absorbent resin (see Patent Publication 1); a method including the steps of polymerizing first-step monomers to form a water-absorbent resin, again adding the monomer to a polymerization reaction mixture in which polymer particles are suspended to polymerize therewith, thereby agglomerating the first-step polymer particles (Patent Publication 2); a method including the steps of polymerizing first-step monomers to form a water-absorbent resin, adding a specified surfactant having HLB of 7 or more to a polymerization reaction mixture in which polymer particles are suspended, again adding the monomer thereto to polymerize, thereby obtaining a large water-absorbent resin (see Patent Publication 3); a method including the steps of polymerizing first-step monomers to form a water-absorbent resin, again adding the monomer to a polymerization reaction mixture in which polymer particles are suspended in the presence of an inorganic powder to polymerize, thereby obtaining a large water-absorbent resin (see Patent Publication 4); and the like have been proposed.

PRIOR ART REFERENCES

PATENT PUBLICATIONS

Patent Publication 1: Japanese Patent Laid-Open No. Hei-3-227301
Patent Publication 2: Japanese Patent Laid-Open No. Hei-5-017509
Patent Publication 3: Japanese Patent Laid-Open No. Hei-9-012613
Patent Publication 4: Japanese Patent Laid-Open No. Hei-9-077810

SUMMARY OF THE INVENTION

PROBLEMS TO BE SOLVED BY THE INVENTION

The water-absorbent resins produced by the methods described in Patent Publications 1 to 4 did not have sufficiently satisfactory flow-through property, even though resins having large particle sizes are obtained. Therefore, an object of the present invention is to provide a method for obtaining a water-absorbent resin having excellent flow-through property, and a water-absorbent resin obtained thereby, and an absorbent material and an absorbent article using the water-absorbent resin.

MEANS TO SOLVE THE PROBLEMS

Specifically, the gist of the present invention relates to:

[1] a method for producing a water-absorbent resin including the step of subjecting primary particles obtained by a first-step reversed phase suspension polymerization to agglomeration according to a second-step reversed phase suspension polymerization, each using an internal-crosslinking agent-added water-soluble ethylenically unsaturated monomer, characterized in that A and B satisfy the relationships of:

$A \leq 5.0 \times 10^{-3}$, and $2 \leq B/A \leq 10$,

wherein an amount of the internal-crosslinking agent added in the first-step monomers, based on 100 mol of the water-soluble ethylenically unsaturated monomer used in the first step, is defined as A mol, and an amount of the internal-crosslinking agent added in the second-step monomers, based on 100 mol of the water-soluble ethylenically unsaturated monomer used in the second step, is defined as B mol;
[2] a water-absorbent resin obtained by the method as defined in the above [1];
[3] an absorbent material containing the water-absorbent resin as defined in the above [2] and a hydrophilic fiber.
[4] an absorbent article comprising the absorbent material as defined in the above [3], held between a liquid-permeable sheet and a liquid-impermeable sheet

EFFECTS OF THE INVENTION

According to the method of the present invention, a water-absorbent resin having excellent flow-through property, an absorbent material and an absorbent article containing the water-absorbent resin can be provided.

BRIEF DESCRIPTION OF THE DRAWINGS

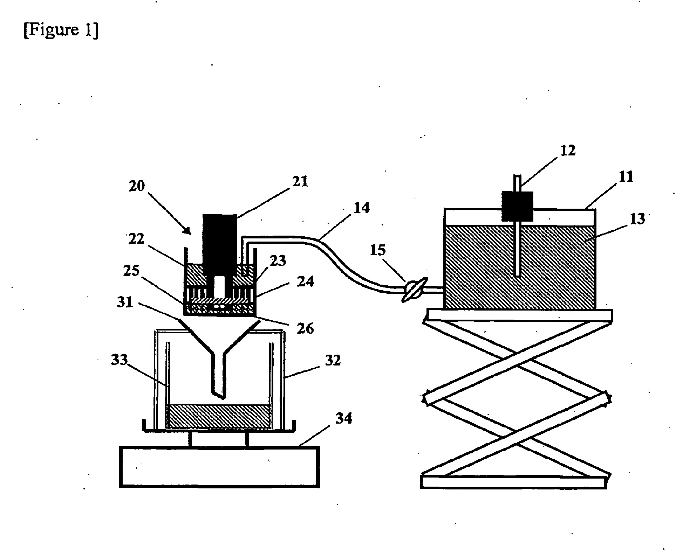

[Figure 1] A schematic view showing an outline constitution of a measurement apparatus for a flow-through rate of an aqueous 0.69% by mass sodium chloride solution.

## MODES FOR CARRYING OUT THE INVENTION

### 1. First-Step Reversed Phase Suspension Polymerization

The method of the present invention is carried out by two-step reversed phase suspension polymerization. A first-step reversed phase suspension polymerization will be explained hereinbelow.

In the first-step, first, a water-soluble ethylenically unsaturated monomer to which an internal-crosslinking agent is added is subjected to a first-step reversed phase suspension polymerization with a radical polymerization initiator in a petroleum hydrocarbon dispersion medium in the presence of a dispersion stabilizer.

The water-soluble ethylenically unsaturated monomer used includes, for example, (meth)acrylic acid and/or salts thereof (In the present specification, "acryl-" and "methacryl-" are together expressed as "(meth)acryl-"; hereinafter referred to the same), 2-(meth)acrylamide-2-methylpropanesulfonic acid and/or salts thereof, nonionic monomers such as (meth)acrylamide, N,N-dimethyl (meth)acrylamide, 2-hydroxyethyl (meth)acrylate, N-methylol (meth)acrylamide, and polyethylene glycol mono(meth)acrylate; and amino group-containing unsaturated monomers such as N,N-diethylaminoethyl (meth)acrylate, N,N-diethylaminopropyl (meth)acrylate, and N,N-diethylaminoethyl (meth)acrylamide, and a quaternary compound thereof; and the like, and at least one member selected from the group of these monomers can be used. Among these water-soluble unsaturated monomers, (meth)acrylic acid or salts thereof, (meth)acrylamide, and N,N-dimethylacrylamide are preferred, and (meth)acrylic acid or salts thereof, and acrylamide are more preferred.

The water-soluble ethylenically unsaturated monomer can be usually used in the form of an aqueous solution. The concentration of the monomer in the aqueous monomer solution is preferably 20% by mass or higher and a saturated concentration or lower, more preferably from 25 to 70% by mass, and even more preferably from 30 to 55% by mass.

In a case where the water-soluble ethylenically unsaturated monomer has an acid group, as in the case of (meth)acrylic acid or 2-(meth)acrylamide-2-methylpropanesulfonic acid, the acid group may be previously neutralized with an alkaline neutralizing agent. The alkaline neutral agent as mentioned above includes aqueous solutions of sodium hydroxide, potassium hydroxide, ammonia, and the like. These alkaline neutralizing agents may be used alone or in combination of two or more kinds.

The neutralization degree in the entire acid groups of the water-soluble ethylenically unsaturated monomer with the alkaline neutralizing agent is preferably with the range of from 10 to 100% by mol, and more preferably within the range of 30 to 80% by mol, from the viewpoint of increasing an osmotic pressure of the resulting water-absorbent resin, thereby increasing water-absorbent capacity, and allowing not to cause some disadvantages in safety or the like by the presence of an excess alkaline neutralizing agent.

The petroleum hydrocarbon dispersion medium includes, for example, aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; aromatic hydrocarbons such as benzene, toluene, and xylene; and the like. These petroleum hydrocarbon dispersion media may be used alone or in combination of two or more kinds. Among these petroleum hydrocarbon dispersion media, n-hexane, n-heptane, and cyclohexane are preferably used, because these dispersion media are readily industrially available, stable in quality and inexpensive. In addition, from the same viewpoint, as an example of the above-mentioned combination of petroleum hydrocarbon dispersion medium, a commercially available Exxsol heptane (manufactured by ExxonMobile, containing 75-85% by mass n-heptane and isomers) is preferred.

The petroleum hydrocarbon dispersion medium is usually used in an amount of preferably from 100 to 1200 parts by mass, and more preferably from 200 to 1000 parts by mass, based on 100 parts by mass of the water-soluble ethylenically unsaturated monomer used in the first-step polymerization, from the viewpoint of homogeneously dispersing a water-soluble ethylenically unsaturated monomer, thereby facilitating control of a polymerization temperature.

As the dispersion stabilizer, a surfactant may be used. The surfactant includes, for example, sucrose fatty acid esters, polyglycerol fatty acid esters, sorbitan fatty acid esters, polyoxyethylene sorbitan fatty acid esters, polyoxyethylene glycerol fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallylformaldehyde condensed polyoxyethylene ethers, polyoxyethylene polyoxypropylene block copolymer, polyoxyethylene polyoxypropylene alkyl ethers, polyethylene glycol fatty acid esters, alkyl glucosides, N-alkyl gluconamides, polyoxyethylene fatty acid amides, polyoxyethylene alkylamines, phosphoric esters of polyoxyethylene alkyl ethers, phosphoric esters of polyoxyethylene alkylallyl ethers, and the like.

Among them, sorbitan fatty acid esters, polyglycerol fatty acid esters and sucrose fatty acid esters are preferred, from the viewpoint of dispersion stability of the monomer. These surfactants may be used alone or in combination of two or more kinds.

In addition, as the dispersion stabilizer, a polymeric dispersion agent may be used together with a surfactant. The polymeric dispersion agent to be used includes, for example, maleic anhydride-modified polyethylene, maleic anhydride-modified polypropylene, maleic anhydride-modified ethylene-propylene copolymer, maleic anhydride-modified EPDM (ethylene-propylene-diene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, ethyl hydroxyethyl cellulose, and the like.

Among these polymeric dispersion agents, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are preferred, from the viewpoint of dispersion stability of the monomer. These polymeric dispersion agents may be used alone or in combination of two or more kinds.

The dispersion stabilizer is used in an amount of preferably from 0.1 to 5 parts by mass, and more preferably from 0.2 to 3 parts by mass, based on the amount of 100 parts by mass of the water-soluble ethylenically unsaturated monomer used in the first-step polymerization, in order to keep an excellent dispersion state of the aqueous solution of the monomer in the petroleum hydrocarbon dispersion medium, and to obtain a dispersion effect accounting to the amount used.

The radical polymerization initiator includes, for example, persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; azo compounds such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazolin-2-yl]propane}dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid); and the like.

Among them, potassium persulfate, ammonium persulfate, sodium persulfate, and 2,2'-azobis(2-amidinopropane)dihydrochloride are preferable, from the viewpoint of being industrially easily available and easily handled. These radical polymerization initiators may be used alone or in combination of two or more kinds.

The radical polymerization initiator is usually used in an amount of preferably from 0.005 to 1 mol, based on 100 mol of the water-soluble ethylenically unsaturated monomer used in the first-step polymerization. When the amount of the radical polymerization initiator used is less than 0.005 mol, there is a risk of requiring a large amount of time for the polymerization reaction. When the amount of the radical polymerization initiator used exceeds 1 mol, there is a risk of causing a sudden polymerization reaction.

Here, the above-mentioned radical polymerization initiator can be used as a redox polymerization initiator together with a reducing agent such as sodium sulfite, sodium hydrogensulfite, ferrous sulfate, or L-ascorbic acid.

The reaction temperature for the polymerization reaction differs depending upon the radical polymerization initiator used. Usually, the reaction temperature is preferably from 20° to 110°C and more preferably from 40° to 90°C. When the reaction temperature is lower than 20°C, the polymerization rate is delayed and the polymerization time is extended, thereby making it economically disadvantageous. When the reaction temperature is higher than 110°C, there is a risk that it would be difficult to remove heat of polymerization, thereby making it difficult to carry out the reaction smoothly. The reaction time is preferably from 0.1 to 4 hours.

As the internal-crosslinking agent to be added to the above-mentioned monomer, for example, a compound having two or more polymerizable unsaturated groups is used. The internal-crosslinking agent includes, for example, di- or tri(meth)acrylic ester of polyols such as (poly)ethylene glycol [in the present specification, for example, "polyethylene glycol" and "ethylene glycol" may be together expressed as "(poly)ethylene glycol"; hereinafter referred to the same], (poly)propylene glycol, trimethylolpropane, glycerol polyoxyethylene glycol, polyoxypropylene glycol, and (poly)glycerol; unsaturated polyesters obtained by reacting the above-mentioned polyols with unsaturated acids such as maleic acid and fumaric acid; bisacrylamides such as N,N'-methylenebisacrylamide; di- or tri(meth)acrylate esters obtained by reacting a polyepoxide with (meth)acrylic acid; carbamyl esters of di(meth)acrylic acid obtained by reacting a polyisocyanate such as tolylene diisocyanate or hexamethylene diisocyanate with hydroxyethyl (meth)acrylate; allylated starch, allylated cellulose, diallyl phthalate, N,N',N"-triallyl isocyanurate, divinylbenzene, and the like.

In addition, as the internal-crosslinking agent, in addition to the above-mentioned compound having two or more polymerizable unsaturated groups, a compound having two or more reactive functional groups can be used. The compound includes, for example, glycidyl group-containing compounds, such as (poly)ethylene glycol diglycidyl ether, (poly)propylene glycol diglycidyl ether, and (poly)glycerol diglycidyl ether; (poly)ethylene glycol, (poly)propylene glycol, (poly)glycerol, pentaerythritol, ethylenediamine, polyethyleneimine, and glycidyl (meth)acrylate, and the like.

These internal-crosslinking agents may be used alone or in combination of two or more kinds.

Among these internal-crosslinking agents, (poly)ethylene glycol diglycidyl ether, (poly)propylene glycol diglycidyl ether, (poly)glycerol diglycidyl ether, and N,N'-methylenebis(meth)acrylamide are preferred, from the viewpoint of

5

having excellent reactivity at low temperatures.

One of the features in the present invention resides in that the amount A mol of the internal-crosslinking agent to be added based on (100 mol of) the monomer used in the first-step reversed phase suspension polymerization is $5.0 \times 10^{-3}$ mol or less, based on 100 mol of the water-soluble ethylenically unsaturated monomer used in the first-step polymerization. The amount A mol is preferably $4.8 \times 10^{-3}$ mol or less, and more preferably $4.7 \times 10^{-3}$ mol or less. Further, the amount A mol is preferably $0.9 \times 10^{-3}$ mol or more, more preferably $1.0 \times 10^{-3}$ mol or more, even more preferably $1.4 \times 10^{-3}$ mol or more, and still even more preferably $1.6 \times 10^{-3}$ mol or more. Accordingly, the amount A mol is preferably within the range of from 0.9 to $5.0 \times 10^{-3}$ mol, more preferably within the range of from 1.0 to $5.0 \times 10^{-3}$ mol, even more preferably within the range of from 1.4 to $4.8 \times 10^{-3}$ mol, and still even more preferably within the range of from 1.6 to $4.7 \times 10^{-3}$ mol.

Although the reasons why a water-absorbent resin having excellent flow-through property is obtained by producing under the conditions as described above are not elucidated, it is presumably based on the following reasons.

When the amount A mol of the first-step internal-crosslinking agent exceeds $5.0 \times 10^{-3}$ mol, the expansion of the primary particles of the polymer obtained by the first-step reversed phase suspension polymerization upon liquid absorption is inhibited by internal-crosslinking, so that the monomer to be added during the second-step polymerization is less likely to be able to be sufficiently absorbed. As a result, the monomer remaining unabsorbed would form a water-absorbent resin in fine powder form that causes gel blocking phenomenon by a second-step reversed phase suspension polymerization, thereby lowering the flow-through property.

In addition, in order to control the water-absorbent properties of the water-absorbent resin, a chain transfer agent may be added thereto. The chain transfer agent as mentioned above can be exemplified by hypophosphites, thiols, thiolic acids, secondary alcohols, amines, and the like.


2. Second-Step Reversed Phase Suspension Polymerization

Next, a second-step reversed phase suspension polymerization will be explained. In the second-step reversed suspension polymerization, to a slurry dispersed with primary particles obtained by the first-step reversed phase suspension polymerization is added an internal-crosslinking agent-added water-soluble ethylenically unsaturated monomer to carry out a polymerization reaction of the monomers. By the reaction, the primary particles dispersed in the slurry are agglomerated.

In the second-step reversed phase suspension polymerization, prior to carrying out the polymerization reaction of the monomers, the suspension polymerization may include the step of cooling a slurry obtained in the first-step reverse phased suspension polymerization to precipitate a dispersion stabilizer contained in the slurry. The cooling temperature is not particularly limited, and the cooling temperature may be usually set at 10° to 50°C or so. The precipitation of the dispersion stabilizer may be confirmed by white turbidity of the slurry; for example, the turbidity may be confirmed by a means such as visual observation, a turbidity gauge, or the like.

As to the kinds, the degree of neutralization, the salts formed by neutralization, and the concentration of the aqueous monomer solution of the water-soluble ethylenically unsaturated monomers used in the second step, it is preferable that those kinds and numerical ranges described in the explanation of the water-soluble ethylenically unsaturated monomer of the first step are employed. In that case, the water-soluble ethylenically unsaturated monomer used in the first-step polymerization can be read as the water-soluble ethylenically unsaturated monomer used in the second-step polymerization. Also, in this case, the kinds and numerical values may be the same as or different from those of the first step.

The water-soluble ethylenically unsaturated monomer of the second step is added in an amount of preferably from 80 to 200 parts by mass, and more preferably from 100 to 160 parts by mass, based on 100 parts by mass of the water-soluble ethylenically unsaturated monomer of the first step, from the viewpoint of obtaining a water-absorbent resin having excellent flow-through property.

The radical polymerization initiator to be added to the water-soluble ethylenically unsaturated monomer of the second step and the amount used can be properly selected and used from those exemplified as the radical polymerization initiator used in the first-step polymerization and the amount used. In that case, the radical polymerization initiator used in the first-step polymerization can be read as the radical polymerization initiator used in the second-step polymerization. Also, in this case, the kinds and the numerical values may be the same or different from those of the first step.

Although the reaction temperature in the second-step reversed phase suspension polymerization also differs depending upon the radical polymerization initiator used, and usually the reaction temperature is preferably from 20° to 110°C, and more preferably from 40° to 90°C.

In addition, the internal-crosslinking agent to be added to the water-soluble ethylenically unsaturated monomer of the second step can be also selected from those exemplified in the first-step polymerization. Here, the internal-crosslinking agent used in the first-step polymerization can be read as the internal-crosslinking agent used in the second-step polymerization. In addition, in this case, the kinds may be the same as or different from those of the first step. Also, a chain transfer agent may be added.

One of the features in the present invention is in that the amount B mol of the internal-crosslinking agent used in the second-step polymerization to be added per (100 mol of) the monomer used in the second step satisfies the relation in connection to the above A mol of $2 \leq B/A \leq 10$. The range for B/A is preferably $3 \leq B/A \leq 9$, and more preferably $3 \leq B/A \leq 8$.

Although the reasons why a water-absorbent resin having excellent flow-through property is obtained by carrying out the method as described above are not elucidated, it is deduced based on the following reasons.

The water-absorbent resin obtained by reversed phase suspension polymerization of the present invention is obtained by agglomerating primary particles. When the relation of the amount B mol of the internal-crosslinking agent of the second step to the amount A mol of the internal-crosslinking agent of the first step, i.e. B/A, is less than 2, the strength of inhibiting the expansion of the particles is weak, so that the gaps between the particles are easily clogged by the expansion of the particles upon water absorption, thereby making it likely to lower the flow-through property. On the other hand, when B/A exceeds 10, the agglomerating force becomes too strong, so that the gaps between the particles become small due to binding of the particles with each other, and that the gaps between the particles are easily clogged by the expansion of the particles upon water absorption, thereby making it likely to lower the flow-through property.

In the present invention, during a period from after the second-step polymerization of the water-soluble ethylenically unsaturated monomer to drying step, it is preferable that a post-crosslinking agent is added to subject the polymer to a post-crosslinking treatment. By the treatment, the water-absorbent resin has an increased crosslinking density near the surface thereof, whereby a water-absorbent resin having increased properties in water absorption capacity under a load, water absorption rate, and gel strength, which are suitable for hygienic material applications can be obtained.

The post-crosslinking agent as mentioned above includes compounds having two or more reactive functional groups. Examples thereof include diglycidyl group-containing compounds such as (poly)ethylene glycol diglycidyl ether, (poly)glycerol (poly)glycidyl ether, (poly)propylene glycol diglycidyl ether, and (poly)glycerol diglycidyl ether; (poly)ethylene glycol, (poly)propylene glycol, (poly)glycerol, pentaerythritol, ethylenediamine, and polyethyleneimine, and the like. Among these post-crosslinking agents, (poly)ethylene glycol diglycidyl ether, (poly)propylene glycol diglycidyl ether, and (poly)glycerol diglycidyl ether are preferred. These post-crosslinking agents may be used alone or in combination of two or more kinds.

The post-crosslinking agent is added in an amount within the range of preferably from 0.005 to 1 mol, and within the range of more preferably from 0.01 to 0.5 mol, based on 100 mol of a total amount of the water-soluble ethylenically unsaturated monomer used in the polymerization, from the viewpoint of increasing crosslinking density near the surface of the water-absorbent resin without lowering the water absorption capacity of the resulting water-absorbent resin, thereby enhancing various properties.

The timing of addition of the post-crosslinking agent may be any time after the termination of the polymerization, without being particularly limited. The post-crosslinking agent is preferably added in the presence of water in an amount within the range of from 1 to 400 parts by mass, more preferably in the presence of water in an amount of from 5 to 200 parts by mass, and even more preferably in the presence of water in an amount of from 10 to 100 parts by mass, based on 100 parts by mass of a total amount of the water-soluble ethylenically unsaturated monomer used for obtaining the water-absorbent resin. As described above, by controlling the amount of water upon addition of the post-crosslinking agent, the crosslinking can be provided more suitably near the surface of the water-absorbent resin, and whereby consequently excellent water absorption capacity can be accomplished.

When a post-crosslinking agent is added, the post-crosslinking agent may be added directly, or may be added in the form of an aqueous solution. Also, a hydrophilic organic solvent may be used, as occasion demands, as a solvent. This hydrophilic organic solvent includes, for example, lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; sulfoxides such as dimethyl sulfoxide; and the like. These hydrophilic organic solvents may be used alone, or in a combination of two or more kinds, or in a mixed solvent with water.

The temperature during the post-crosslinking reaction is preferably from 50° to 250°C, more preferably from 60° to 180°C, even more preferably from 60° to 140°C, and still even more preferably from 70° to 120°C.

In the present invention, the drying step may be carried out at a normal pressure or under a reduced pressure, or the drying step may be carried under a gas stream such as nitrogen, in order to increase drying efficiency. In a case where the drying step is carried out at a normal pressure, the drying temperature is preferably from 70° to 250°C, more preferably from 80° to 180°C, even more preferably from 80° to 140°C, and still even more preferably from 90° to 130°C. In addition, in a case where the drying step is carried under a reduced pressure, the drying temperature is preferably from 60° to 100°C, and more preferably from 70° to 90°C.

The water-absorbent resin according to the present invention thus obtained has excellent flow-through property, so that the water-absorbent resin is suitably used in an absorbent material and an absorbent article using the absorbent material.

3. Water-Absorbent Resin

The water percentage of the water-absorbent resin is preferably 20% by mass or less, and more preferably 10% by mass or less, from the viewpoint of keeping fluidity. In addition, in order to improve fluidity, an amorphous silica powder may be added to the water-absorbent resin.

The water-absorbent resin has a water-retention capacity of saline solution of preferably 32 g/g or less, and more preferably from 27 to 31 g/g, from the viewpoint of having excellent flow-through property after absorbing a liquid to allow the resin to be swollen, and having a high absorption capacity. The water-retention capacity of saline solution of the water-absorbent resin is a value obtained according to a measurement method described in EXAMPLES set forth below.

In the present specification, the flow-through property of the water-absorbent resin is expressed as a flow-through rate of an aqueous 0.69% by mass sodium chloride solution. The flow-through rate of an aqueous 0.69% by mass sodium chloride solution of the water-absorbent resin is preferably 100 [g/10 minutes] or more, and more preferably 120 [g/10 minutes] or more, from the viewpoint of increasing properties such as liquid permeation time and liquid diffusibility in an absorbent material using the water-absorbent resin. The flow-through rate of an aqueous 0.69% by mass sodium chloride solution of the water-absorbent resin is a value obtained according to a measurement method described in EXAMPLES set forth below.

The water-absorbent resin has a median particle size of preferably from 200 to 600 $\mu$m, more preferably from 250 to 500 um, and even more preferably from 300 to 450 $\mu$m. The water-absorbent resin having the defined median particle size is suitable for an absorbent material and an absorbent article using the absorbent material. The median particle size of the water-absorbent resin is a value obtained according to a measurement method described in EXAMPLES set forth below.

4. Absorbent Material and Absorbent Article

The absorbent material using the water-absorbent resin according to the present invention comprises a water-absorbent resin and a hydrophilic fiber. The construction of the absorbent material includes, for example, a mixed dispersion obtained by mixing a water-absorbent resin and a hydrophilic fiber in a homogeneous composition, a sandwich structure in which a water-absorbent resin is sandwiched between layered hydrophilic fibers, a construction in which a water-absorbent resin and a hydrophilic fiber are wrapped around tissue paper, and the like. The present invention is not limited to those exemplified above.

Other components, for example, adhesive binders for increasing shape retention of the absorbent material, such as heat-fusible synthetic fibers, hot melt adhesives, and adhesive emulsions may be added to the absorbent material.

The hydrophilic fiber includes, for example, cellulose fibers such as cotton pulps, mechanical pulps, chemical pumps, semi-chemical pulps, and the like obtained from wood; artificial cellulose fibers such as rayon and acetate; fibers made of synthetic resins such as hydrophilically treated polyamide, polyesters, and polyolefins; and the like.

The absorbent article using the water-absorbent resin according to the present invention has a structure in which the above absorbent material is held between a liquid-permeable sheet and a liquid-impermeable sheet.

The liquid-permeable sheet includes, for example, air-through, spun-bond, chemical bond, or needle punched nonwoven fabrics made of fibers of a polyethylene, a polypropylene, a polyester, or the like.

The liquid-impermeable sheet includes, for example, synthetic resin films made of a resin such as a polyethylene, a polypropylene or a polyvinyl chloride.

The kinds of the absorbent articles are not particularly limited. Representative examples thereof include, for example, hygienic materials such as disposable diapers, sanitary napkins, and incontinence pads; urine-absorbent materials for pets; materials for civil engineering and construction such as packing materials; food freshness retaining materials such as drip absorbents and cold-reserving agents; horticultural articles such as water-retaining materials for soils; and the like.

EXAMPLES

Water content, water-retention capacity of saline solution, median particle size, and flow-through rate of an aqueous 0.69% by mass sodium chloride solution of the water-absorbent resin obtained in each of Examples and Comparative Examples were evaluated by the methods shown hereinbelow.

< Water Content >

About 2 grams of a water-absorbent resin was accurately measured, i.e. Wa (g), in an aluminum foil case (No. 8) of which mass was previously measured. The above sample was dried for 2 hours with a hot-air dryer (manufactured by ADVANTEC), the internal temperature of which was set to 105°C. Thereafter, the dried sample was allowed to cool in a desiccator, and the mass Wb (g) of the water-absorbent resin after drying was measured. The water content of the water-absorbent resin was calculated from the following formula:

$$\text{Water Content (\%)} = [\text{Wa} - \text{Wb}]/\text{Wa} \times 100$$

< Water-Retention Capacity of Saline Solution >

Five-hundred grams of an aqueous 0.9% by mass sodium chloride solution (saline solution) was weighed in a 500-mL beaker. The amount 2.0 g of a water-absorbent resin was added thereto, while stirring the solution at a rate of 600 r/min, to disperse so as not to cause an unswollen lump of the water-absorbent resin. Under the state of stirring, the dispersion was allowed to stand for 30 minutes, to sufficiently make the water-absorbent resin swollen. Thereafter, the dispersion was poured into a cotton bag (Cotton Broadcloth No. 60, width 100 mm × length 200 mm), and an upper part of the cotton bag was tied up with a rubber band. The cotton bag was dehydrated for 1 minute with a dehydrator (manufactured by Kokusan Enshinki Co., Ltd., product number: H-122) set to have a centrifugal force of 167 G. The mass Wc (g) of the cotton bag containing the swollen gel after dehydration was measured. The same procedures were carried out without adding a water-absorbent resin, and the empty mass Wd (g) of the cotton bag upon wetting was measured. The water-retention capacity was calculated from the following formula:

$$\text{Water-Retention Capacity of Saline Solution (g/g)}$$

$$= [\text{Wc} - \text{Wd}]\ (\text{g})/\text{Mass of Water-Absorbent Resin (g)}$$

< Median Particle Size >

With 50 g of a water-absorbent resin was mixed as a lubricant 0.25 g of amorphous silica (Sipemat 200, Degussa Japan, K.K.), to give a water-absorbent resin for the measurement.

JIS standard sieves, a sieve having an opening of 850 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 150 $\mu$m, and a receiving tray were combined in order from the top. The above water-absorbent resin for the measurement was placed on an uppermost sieve of the combined sieves, and shaken for 20 minutes with a rotating and tapping shaker machine to classify the resin.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a median particle size by joining the plots on the probability paper in a straight line.

< Flow-Through Rate of Aqueous 0.69% by Mass Sodium Chloride Solution >

(a) Preparation of Artificial Urine

Two grams of potassium chloride, 2 g of anhydrous sodium sulfate, 0.19 g of calcium chloride, 0.23 g of magnesium chloride, 0.85 g of ammonium dihydrogenphosphate, 0.15 g of ammonium hydrogenphosphate, and a proper amount of distilled water were placed in a 1-L container to completely dissolve the components. Distill water was further added thereto to adjust the volume of the entire aqueous solution to 1 L.

(b) Setting of Measurement Apparatus

As a measurement apparatus, one having an outline constitution shown in Figure 1 was used. The apparatus comprised a tank 11 equipped with a glass tube 12 for static pressure adjustment, in which a lower end of the glass tube 12 is so arranged that a height of a liquid interface inside a cylinder 22 of an aqueous 0.69% by mass sodium chloride solution 13 could be maintained at a height 5 cm from the bottom of a swollen gel 25. The aqueous 0.69% by mass sodium chloride solution 13 in the tank 11 is supplied to the inside of the cylinder 22 through an L-shaped tube 14 equipped with a cock. A container 33 for collecting a passed liquid is arranged below the cylinder 22, and the collecting container 33 was placed on balance and scales 34. The cylinder 22 had an inner diameter of 6 cm, a bottom of a lower part of which was provided with No. 400 stainless steel wire gauze (sieve opening 38 μm) 26. A lower part of a piston-style weight 21 is provided with holes 23 sufficient to allow liquid permeation, and a glass filter 24 having excellent transmittance was provided at the bottom thereof so that a water-absorbent resin or a swollen gel thereof is not entered into the holes 23.

(c) Measurement of Flow-Through Rate

In a cylindrical container 20 was evenly placed 0.9 g of a water-absorbent resin, and the water-absorbent resin was allowed to swell for 60 minutes in a synthetic urine under a load of 2.07 kPa using a piston-style weight 21, to form a swollen gel 25.
Next, under a load of 2.07 kPa, an aqueous 0.69% by mass sodium chloride solution 13 was supplied from the tank 11 to a swollen gel 25 at a constant static pressure capable of maintaining a height of a liquid interface inside the cylinder 22 of the aqueous solution 13 to a height 5 cm above the bottom of the swollen gel 25.
The mass of the aqueous solution 13 entering the collecting container 33 after passing through the swollen gel 25 over a period of 10 minutes from the beginning of the supplying of the aqueous solution 13 was measured, and defined as a flow-through rate ([g/10 minutes]). This measurement was carried out at room temperature (20° to 25°C).

[Example 1]

A 2-L cylindrical round bottomed separable flask equipped with a stirrer, double-paddle blades, a reflux condenser, a dropping funnel, and a nitrogen gas inlet tube was furnished. This flask was charged with 340 g of n-heptane, and 0.92 g of a sucrose stearate (manufactured by Mitsubishi-Kagaku Foods Corporation, Ryoto sugar ester S-370) and 0.92 g of a maleic anhydride-modified ethylene-propylene copolymer (manufactured by Mitsui Chemicals, Inc., Hi-wax 1105A) were added thereto as dispersion stabilizers. The temperature was raised to 80°C to dissolve the surfactant, and thereafter the solution was cooled to 50°C.
On the other hand, a 500 mL-Erlenmeyer flask was charged with 92 g (1.02 mol) of an 80% by mass aqueous solution of acrylic acid, and 146.0 g of a 21 % by mass aqueous sodium hydroxide was added dropwise thereto with cooling from external to neutralize 75% by mol. Thereafter, 0.11 g (0.41 mmol) of potassium persulfate as a radical polymerization initiator, and 1.6 mg ($0.09 \times 10^{-1}$ mmol, corresponding to $0.9 \times 10^{-3}$ mol per 100 mol of the monomers for the first step) of ethylene glycol diglycidyl ether as an internal-crosslinking agent were added thereto to dissolve, to prepare an aqueous monomer solution for the first step.
An entire amount of the above-mentioned aqueous monomer solution for the first step was added to the above-mentioned separable flask, and the internal of the system was sufficiently replaced with nitrogen. Thereafter, the flask was immersed and heated in a water bath kept at 70°C, and a first-step polymerization was carried out for 30 minutes, to give a reaction mixture for the first-step polymerization.
On the other hand, another 500 mL-Erlenmeyer flask was charged with 128.8 g (1.43 mol) of an 80% by mass aqueous solution of acrylic acid, and 159.0 g of a 27% by mass aqueous sodium hydroxide was added dropwise thereto with cooling from external to neutralize 75% by mol. Thereafter, 0.16 g (0.59 mmol) of potassium persulfate as a radical polymerization initiator, and 0.02 g ($1.15 \times 10^{-1}$ mmol, corresponding to $8.0 \times 10^{-3}$ mol per 100 mol of the monomers for the second step) of ethylene glycol diglycidyl ether as an internal-crosslinking agent were added thereto to dissolve, to prepare an aqueous monomer solution for the second step.
The above-mentioned aqueous monomer solution for the first step was then cooled to 22°C. The aqueous monomer solution for the second step mentioned above at the same temperature was added to the internal of the system to allow the aqueous monomer solution to be absorbed for 30 minutes and at the same time the internal of the system was sufficiently replaced with nitrogen. Thereafter, the flask was again immersed in a water bath at 70°C, the temperature was raised, and a second-step polymerization was carried out for 30 minutes.
After the second-step polymerization, the reaction mixture was heated with an oil bath at 125°C, and n-heptane and water were subjected to azeotropic distillation to remove 220 g of water to the external of the system, while refluxing n-heptane. Thereafter, 8.17 g (0.94 mmol) of a 2% aqueous solution of ethylene glycol diglycidyl ether was added thereto, and a post-crosslinking reaction was carried out at 80°C for 2 hours. Subsequently, the reaction mixture was heated with an oil bath kept at 125°C, and n-heptane was evaporated to dryness, to give 228.2 g of a water-absorbent resin (A). The resulting water-absorbent resin had a median particle size of 380 μm and a water content of 7.1%. The measurement results for each of the properties are as shown in Table 1.

[Example 2]

The same procedures as in Example 1 were carried out except that the amount of the internal-crosslinking agent for the first step was changed to 2.8 mg ($0.16 \times 10^{-1}$ mmol, corresponding to $1.6 \times 10^{-3}$ mol per 100 mol of the monomers for the first step), and that the amount of the internal-crosslinking agent for the second step was changed to 0.03 g ($1.72 \times 10^{-1}$ mmol, corresponding to $12.0 \times 10^{-3}$ mol per 100 mol of the monomers for the second step), to give 230.1 g of a water-absorbent resin (B). The resulting water-absorbent resin had a median particle size of 370 $\mu$m and a water content of 7.6%. The measurement results for each of the properties are as shown in Table 1.

[Example 3]

The same procedures as in Example 1 were carried out except that the internal-crosslinking agent was changed to N,N'-methylenebisacrylamide, that the amount of the internal-crosslinking agent for the first step was changed to 4.6 mg ($0.30 \times 10^{-1}$ mmol, corresponding to $2.9 \times 10^{-3}$ mol per 100 mol of the monomers for the first step), and that the amount of the internal-crosslinking agent for the second step was changed to 0.03 g ($1.95 \times 10^{-1}$ mmol, corresponding to $13.6 \times 10^{-3}$ mol per 100 mol of the monomers for the second step), to give 229.5 g of a water-absorbent resin (C). The resulting water-absorbent resin had a median particle size of 350 $\mu$m and a water content of 7.5%. The measurement results for each of the properties are as shown in Table 1.

[Example 4]

The same procedures as in Example 2 were carried out except that the amount of the internal-crosslinking agent for the first step was changed to 8.3 mg ($0.48 \times 10^{-1}$ mmol, corresponding to $4.7 \times 10^{-3}$ mol per 100 mol of the monomers for the first step), to give 227.9 g of a water-absorbent resin (D). The resulting water-absorbent resin had a median particle size of 330 $\mu$m and a water content of 7.3%. The measurement results for each of the properties are as shown in Table 1.

[Example 5]

The same procedures as in Example 4 were carried out except that the amount of the internal-crosslinking agent for the second step was changed to 0.07 g ($4.02 \times 10^{-1}$ mmol, corresponding to $28.1 \times 10^{-3}$ mol per 100 mol of the monomers for the second step), to give 229.9 g of a water-absorbent resin (E). The resulting water-absorbent resin had a median particle size of 320 $\mu$m and a water content of 7.5%. The measurement results for each of the properties are as shown in Table 1.

[Comparative Example 1]

The same procedures as in Example 5 were carried out except that the amount of the internal-crosslinking agent for the first step was changed to 0.01 g ($0.57 \times 10^{-1}$ mmol, corresponding to $5.6 \times 10^{-3}$ mol per 100 mol of the monomers for the first step), to give 230.4 g of a water-absorbent resin (F). The resulting water-absorbent resin had a median particle size of 280 $\mu$m and a water content of 7.7%. The measurement results for each of the properties are as shown in Table 1.

[Comparative Example 2]

The same procedures as in Example 2 were carried out except that the amount of the internal-crosslinking agent for the second step was changed to 4.0 mg ($0.23 \times 10^{-1}$ mmol, corresponding to $1.6 \times 10^{-3}$ mol per 100 mol of the monomers for the second step), to give 225.8 g of a water-absorbent resin (G). The resulting water-absorbent resin had a median particle size of 380 $\mu$m and a water content of 7.0%. The measurement results for each of the properties are as shown in Table 1.

[Comparative Example 3]

The same procedures as in Example 4 were carried out except that the amount of the internal-crosslinking agent for the second step was changed to 0.01 g ($0.57 \times 10^{-1}$ mmol, corresponding to $4.0 \times 10^{-3}$ mol per 100 mol of the monomers for the second step), to give 227.4 g of a water-absorbent resin (H). The resulting water-absorbent resin had a median particle size of 310 $\mu$m and a water content of 7.2%. The measurement results for each of the properties are as shown in Table 1.

[Comparative Example 4]

The same procedures as in Example 2 were carried out except that the amount of the internal-crosslinking agent for the second step was changed to 0.05 g ($2.87 \times 10^{-1}$ mmol, corresponding to $20.1 \times 10^{-3}$ mol per 100 mol of the monomers for the second step), to give 229.1 g of a water-absorbent resin (I). The resulting water-absorbent resin had a median particle size of 370 $\mu$m and a water content of 7.6%. The measurement results for each of the properties are as shown in Table 1.

[Comparative Example 5]

The same procedures as in Example 4 were carried out except that the amount of the internal-crosslinking agent for the second step was changed to 0.13 g ($7.46 \times 10^{-1}$ mmol, corresponding to $52.2 \times 10^{-3}$ mol per 100 mol of the monomers for the second step), to give 228.5 g of a water-absorbent resin (J). The resulting water-absorbent resin had a median particle size of 310 $\mu$m and a water content of 7.5%. The measurement results for each of the properties are as shown in Table 1.

[Table 1]

Table 1

| | A $\times 10^{-3}$ [mol[a)]] | B/A | Water-Retention Capacity of Saline Solution [g/g] | Flow-Through Rate of Aqueous 0.69% by Mass Sodium Chloride Solution [g/10 minutes] |
|---|---|---|---|---|
| Ex. 1 | 0.9 | 9 | 32.2 | 95 |
| Ex. 2 | 1.6 | 8 | 29.6 | 147 |
| Ex. 3 | 2.9 | 5 | 28.8 | 178 |
| Ex. 4 | 4.7 | 3 | 27.9 | 287 |
| Ex. 5 | 4.7 | 6 | 27.1 | 211 |
| Comp. Ex. 1 | 5.6 | 5 | 26.5 | 73 |
| Comp. Ex. 2 | 1.6 | 1 | 33.4 | 34 |
| Comp. Ex. 3 | 4.7 | 1 | 31.8 | 51 |
| Comp. Ex. 4 | 1.6 | 13 | 28.8 | 65 |
| Comp. Ex. 5 | 4.7 | 11 | 25.8 | 80 |

a) Amount based on 100 mol of water-soluble ethylenically unsaturated monomer.

It is clear from Table 1 that water-absorbent resins having excellent performance in flow-through rates were obtained according to the methods of Examples 1 to 5.

On the other hand, in Comparative Examples, in a case of a method where the amount of an internal-crosslinking agent for the first step is large (Comparative Example 1), the amount of particles in the form of fine powder is large, so that a water-absorbent resin having slower flow-through rate was obtained. In cases where the amounts of an internal-crosslinking agent for the second step are small (Comparative Examples 2 and 3), the gaps between the particles are clogged due to expansion of the particles, so that water-absorbent resins having slower flow-through rates were obtained. In cases where proportions of an internal-crosslinking agent for the second step are large (Comparative Examples 4 and 5), the gaps between the particles are narrow, so that water-absorbent resins having slower flow-through rates were obtained.

Next, absorbent materials and absorbent articles were prepared using the water-absorbent resins obtained in Examples 1 to 5 and Comparative Examples 1 to 5.

[Example 6]

Ten grams of the water-absorbent resin (A) obtained in Example 1 and 10 g of crushed wooden pulp were dry-blended with a mixer, and the mixture was sprayed on tissue paper having sizes of 40 cm × 12 cm and a weight of 1 g. Thereafter, tissue paper having the same sizes and weight was layered from an upper part, and formed into a sheet. A 196 kPa load was applied to the entire sheet for 30 seconds to press the sheet, thereby giving an absorbent material. The resulting absorbent material was sandwiched with a polyethylene air-through, porous liquid-permeable sheet having dimensions of 40 cm × 12 cm and a basis weight of 20 g/m$^2$, and a polyethylene air-through porous liquid-impermeable sheet having the same dimensions and the same basis weight.

[Example 7]

The same procedures as in Example 6 were carried out except that the water-absorbent resin (B) obtained in Example 2 was used, to give an absorbent material and an absorbent article using the absorbent material.

[Example 8]

The same procedures as in Example 6 were carried out except that the water-absorbent resin (C) obtained in Example 3 was used, to give an absorbent material and an absorbent article using the absorbent material.

[Example 9]

The same procedures as in Example 6 were carried out except that the water-absorbent resin (D) obtained in Example 4 was used, to give an absorbent material and an absorbent article using the absorbent material.

[Example 10]

The same procedures as in Example 6 were carried out except that the water-absorbent resin (E) obtained in Example 5 was used, to give an absorbent material and an absorbent article using the absorbent material.

[Comparative Example 6]

The same procedures as in Example 6 were carried out except that the water-absorbent resin (F) obtained in Comparative Example 1 was used, to give an absorbent material and an absorbent article using the absorbent material.

[Comparative Example 7]

The same procedures as in Example 6 were carried out except that the water-absorbent resin (G) obtained in Comparative Example 2 was used, to give an absorbent material and an absorbent article using the absorbent material.

[Comparative Example 8]

The same procedures as in Example 6 were carried out except that the water-absorbent resin (H) obtained in Comparative Example 3 was used, to give an absorbent material and an absorbent article using the absorbent material.

[Comparative Example 9]

The same procedures as in Example 6 were carried out except that the water-absorbent resin (I) obtained in Comparative Example 4 was used, to give an absorbent material and an absorbent article using the absorbent material.

[Comparative Example 10]

The same procedures as in Example 6 were carried out except that the water-absorbent resin (J) obtained in Comparative Example 5 was used, to give an absorbent material and an absorbent article using the absorbent material.

Each of the absorbent articles obtained was evaluated in accordance with the following methods. The results are shown in Table 2.

< Evaluations of Absorbent Articles >

(a) Preparation of Test Solutions

In a 5-L container were placed 30 g of sodium chloride, 0.9 g of calcium chloride dihydrate, 1.8 g of magnesium chloride hexahydrate, and a proper amount of distilled water to completely dissolve. Next, 7.5 g of an aqueous 1% by mass poly(oxyethylene) isooctylphenyl ether solution was added thereto, and distilled water was further added to adjust the mass of the overall aqueous solution to 3000 g. Thereafter, the mixed solution was colored with a small amount of Blue No. 1 to prepare a test solution.

(b) Liquid Permeation Time

An absorbent article was placed on a horizontal table. A cylindrical cylinder having an inner diameter of 3 cm was placed near the central section of this body liquid-absorbent article, and a 50 mL test solution was supplied into the solution at one time. At the same time, a time period until the test solution completely disappeared was measured with a stopwatch, which is referred to as a first liquid permeation time (sec). Next, the above cylinder was removed, the absorbent article was kept in that state, the above cylindrical cylinder was placed at the same position as the first liquid permeation time after 30 minutes and after 60 minutes from the beginning of the supplying of the first test solution, to measure second and third liquid permeation time (sec). A total of the number of seconds for the first to third liquid permeation time is referred to as a total liquid permeation time. The shorter the total liquid permeation time, the more preferred the absorbent article. For example, the total liquid permeation time is preferably 70 seconds or less, and more preferably 65 seconds or less.

(c) Amount of Re-wet

After 60 minutes passed from the termination of the measurement of the liquid permeation time mentioned above, the cylinder was removed, about 80 sheets of filter paper of 10 cm each side, of which mass was previously measured (We (g), about 70 g), were stacked near the liquid feeding position of the body liquid-absorbent article, and a 5 kg weight having a size of 10 cm × 10 cm was placed thereon. After 5 minutes of applying a load, the mass (Wf (g)) of the filter papers was measured, and an increased mass was defined as the amount of re-wet (g) as follows:

$$\text{Amount of Liquid Re-wet (g)} = \text{Wf} - \text{We}$$

The smaller the amount of re-wet, it can be said that it is more preferred as an absorbent article. For example, the amount of re-wet is preferably 30 g or less, and more preferably 28 g or less.

(d) Diffusion Length

The diffusion size (cm) in the longitudinal direction of each of the absorbent articles in which the test solution was permeated was measured within 5 minutes after the measurement of the above amount of re-wet. Here, the numerical figures below the decimal place were rounded off to a nearest whole number. The larger the numerical figures for the diffusion length, the more favorable the diffusibility of the test solution, so that it can be that they are preferred.
[Table 2]

Table 2

| | Resin Used | Liquid Permeation Time [sec] | | | | Amount of Re-wet [g] | Diffusion Length [cm] |
|---|---|---|---|---|---|---|---|
| | | 1 | 2 | 3 | Total | | |
| Ex. 6 | A | 21 | 19 | 28 | 68 | 28.2 | 17 |
| Ex. 7 | B | 22 | 18 | 25 | 65 | 27.1 | 19 |
| Ex. 8 | C | 21 | 17 | 24 | 62 | 27.5 | 19 |
| Ex. 9 | D | 20 | 17 | 20 | 57 | 25.8 | 21 |
| Ex. 10 | E | 20 | 18 | 21 | 59 | 26.8 | 20 |
| Comp. Ex. 6 | F | 22 | 20 | 29 | 71 | 33.1 | 16 |
| Comp. Ex. 7 | G | 23 | 22 | 36 | 81 | 32.6 | 14 |

(continued)

| | Resin Used | Liquid Permeation Time [sec] | | | | Amount of Re-wet [g] | Diffusion Length [cm] |
|---|---|---|---|---|---|---|---|
| | | 1 | 2 | 3 | Total | | |
| Comp. Ex. 8 | H | 22 | 20 | 33 | 75 | 31.8 | 15 |
| Comp. Ex. 9 | I | 21 | 20 | 31 | 72 | 32.4 | 15 |
| Comp. Ex. 10 | J | 21 | 19 | 29 | 69 | 33.5 | 16 |

As is clear from Table 2, it can be seen that the absorbent articles of Examples 6 to 10 in which the water-absorbent resins (A) to (E) having excellent flow-through property were used had short liquid permeation time and small amounts of re-wet.

On the other hand, in Comparative Examples, all of the absorbent articles, including one having a large amount of an internal-crosslinking agent for the first step and lowered performance in flow-through rate (Comparative Example 6), ones having smaller proportions in an internal-crosslinking agent for the second step and lowered performance in flow-through rates (Comparative Examples 7 and 8), and ones having larger proportions in an internal-crosslinking agent for the second step and lowered performance in flow-through rates (Comparative Examples 9 and 10) have worsened performance in flow-through rates and diffusion length of the absorbent articles. Therefore, ones having lowered water-retention capacity of saline solution (Comparative Examples 6 and 10) are more likely to have larger amounts of re-wet.

INDUSTRIAL APPLICABILITY

Since the water-absorbent resin obtained by the method according to the present invention has excellent flow-through property, the water-absorbent resin is especially suitably used as hygienic materials such as thinned sanitary napkins and disposable diapers.

EXPLANATION OF NUMERICAL SYMBOLS

11    tank
12    glass tube for static pressure adjustment
13    aqueous 0.69% by mass sodium chloride solution
14    L-shaped tube with a cock
15    cock
20    cylindrical container
21    piston style weight
22    cylinder
23    hole
24    glass filter
25    swollen gel
26    stainless steel wire gauze
31    funnel
32    support
33    collecting container
34    balance and scales

**Claims**

1. A method for producing a water-absorbent resin comprising subjecting primary particles obtained by a first-step reversed phase suspension polymerization to agglomeration according to a second-step reversed phase suspension polymerization, each step using an internal-crosslinking agent-added water-soluble ethylenically unsaturated monomer,

   **characterized in that** A and B satisfy the relationships of:

$$A \leq 5.0 \times 10^{-3}, \text{ and } 2 \leq B/A \leq 10,$$

wherein an amount of the internal-crosslinking agent added in the first-step monomers, based on 100 mol of the water-soluble ethylenically unsaturated monomer used in the first step, is defined as A mol, and an amount of the internal-crosslinking agent added in the second-step monomers, based on 100 mol of the water-soluble ethylenically unsaturated monomer used in the second step, is defined as B mol.

2. The method for producing a water-absorbent resin according to claim 1, wherein the internal-crosslinking agent is at least one member selected from the group consisting of (poly)ethylene glycol diglycidyl ether, (poly)propylene glycol diglycidyl ether, (poly)glycerol diglycidyl ether, and N,N'-methylenebis(meth)acrylamide.

3. The method for producing a water-absorbent resin according to claim 1 or 2, wherein after the termination of the second-step reversed phase suspension polymerization of the water-soluble ethylenically unsaturated monomer, a post-crosslinking agent is added to carry out post-crosslinking.

4. The method for producing a water-absorbent resin according to any one of claims 1 to 3, wherein water-soluble ethylenically unsaturated monomer is at least one member selected from the group consisting of (meth)acrylic acid or salts thereof, (meth)acrylamide, and N,N-dimethylacrylamide.

5. A water-absorbent resin obtained by the method as defined in any one of claims 1 to 4.

6. An absorbent material comprising the water-absorbent resin as defined in claim 5 and a hydrophilic fiber.

7. An absorbent article comprising the absorbent material as defined in claim 6, held between a liquid-permeable sheet and a liquid-impermeable sheet.

**Patentansprüche**

1. Verfahren zur Herstellung eines wasserabsorbierenden Harzes, umfassend das Aussetzen von durch eine Erstschritt-Umkehrphasen-Suspensionspolymerisation erhaltenen Primärteilchen einer Agglomeration gemäß einer Zweitschritt-Umkehrphasen-Suspensionspolymerisation, wobei jeder Schritt ein wasserlösliches ethylenisch ungesättigtes Monomer mit hinzugefügtem internen Vernetzungsmittel verwendet,
**dadurch gekennzeichnet, daß** A und B den Beziehungen genügen:

$$A \leq 5,0 \times 10^{-3}, \text{ und } 2 \leq B/A \leq 10,$$

wobei eine Menge des internen Vernetzungsmittels, welches zu den Erstschritt-Monomeren hinzugefügt wird, basierend auf 100 mol des im ersten Schritt verwendeten wasserlöslichen ethylenisch ungesättigten Monomers, als A mol definiert ist, und eine Menge des internen Vernetzungsmittels, welches zu den Zweitschritt-Monomeren hinzugefügt wird, basierend auf 100 mol des im zweiten Schritt verwendeten wasserlöslichen ethylenisch ungesättigten Monomers, als B mol definiert ist.

2. Verfahren zur Herstellung eines wasserabsorbierenden Harzes nach Anspruch 1, wobei das interne Vernetzungsmittel mindestens ein Mitglied ist, ausgewählt aus der Gruppe, bestehend aus (Poly)ethylenglycoldiglycidylether, (Poly)propylenglycoldiglycidylether, (Poly)glycerindiglycidylether, und N,N'-Methylenbis(meth)acrylamid.

3. Verfahren zur Herstellung eines wasserabsorbierenden Harzes nach Anspruch 1 oder 2, wobei nach dem Beenden der Zweitschritt-Umkehrphasen-Suspensionspolymerisation des wasserlöslichen ethylenisch ungesättigten Monomers, ein Nach-Vernetzungsmittel hinzugefügt wird, um Nach-Vernetzung durchzuführen.

4. Verfahren zur Herstellung eines wasserabsorbierenden Harzes nach einem der Ansprüche 1 bis 3, wobei das wasserlösliche ethylenisch ungesättigte Monomer mindestens ein Mitglied ist, ausgewählt aus der Gruppe, bestehend aus (Meth)acrylsäure oder Salzen davon, (Meth)acrylamid, und N,N-Dimethylacrylamid.

**5.** Wasserabsorbierendes Harz, erhalten durch das Verfahren wie in einem der Ansprüche 1 bis 4 definiert.

**6.** Absorptionsmaterial, umfassend das wasserabsorbierende Harz wie in Anspruch 5 definiert und eine hydrophile Faser.

**7.** Absorptionsgegenstand, umfassend das Absorptionsmaterial wie in Anspruch 6 definiert, welches zwischen einem flüssigkeitsdurchlässigen Blatt und einem flüssigkeitsundurchlässigen Blatt gehalten wird.

**Revendications**

**1.** Procédé de production d'une résine absorbant l'eau comprenant la soumission de particules primaires obtenues par une première étape de polymérisation d'une suspension de phase inversée à une agglomération selon une deuxième étape de polymérisation de suspension de phase inversée, chaque étape utilisant un agent de réticulation interne additionné d'un monomère éthyléniquement insaturé soluble dans l'eau, **caractérisé en ce que** A et B satisfont les relations suivantes :

$$A \leq 5{,}0 \times 10^{-3}, \text{ et } 2 \leq B/A \leq 10,$$

dans lequel une quantité de l'agent de réticulation interne ajouté dans les monomères de la première étape, sur la base de 100 moles du monomère éthyléniquement insaturé soluble dans l'eau utilisé dans la première étape, est définie comme A moles, et une quantité de l'agent de réticulation interne ajouté dans les monomères de la deuxième étape, sur la base de 100 moles du monomère éthyléniquement insaturé soluble dans l'eau utilisé dans la deuxième étape, est définie comme B moles.

**2.** Procédé de production d'une résine absorbant l'eau selon la revendication 1, dans lequel l'agent de réticulation interne est au moins un élément sélectionné dans le groupe comprenant le (poly)éthylène glycol diglycidyl éther, le (poly)propylène glycol diglycidyl éther, le (poly)glycérol diglycidyl éther et le N,N'-méthylène-bis(méth)acrylamide.

**3.** Procédé de production d'une résine absorbant l'eau selon la revendication 1 ou 2, dans lequel, après la fin de la deuxième étape de polymérisation de suspension de phase inversée du monomère éthyléniquement insaturé soluble dans l'eau, un agent de post-réticulation est ajouté pour exécuter une post-réticulation.

**4.** Procédé de production d'une résine absorbant l'eau selon l'une quelconque des revendications 1 à 3, dans lequel le monomère éthyléniquement insaturé soluble dans l'eau est au moins un élément sélectionné dans le groupe comprenant l'acide (méth)acrylique ou des sels de celui-ci, le (méth)acrylamide et le N,N-diméthylacrylamide.

**5.** Résine absorbant l'eau obtenue par le procédé selon l'une quelconque des revendications 1 à 4.

**6.** Matériau absorbant comprenant la résine absorbant l'eau selon la revendication 5 et une fibre hydrophile.

**7.** Article absorbant comprenant le matériau absorbant selon la revendication 6, maintenu entre une feuille perméable au liquide et une feuille imperméable au liquide.

[Figure 1]

**EP 2 692 741 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI3227301 B
- JP HEI5017509 B
- JP HEI9012613 B
- JP HEI9077810 B